# EUROPÄISCHE PATENTSCHRIFT

(11) **EP 0 776 887 B1**
(45) Veröffentlichungstag und Bekanntmachung des Hinweises auf die Patenterteilung: **29.03.2000**
(21) Anmeldenummer: 96115523.1
(22) Anmeldetag: 27.09.1996
(51) Int. Cl.: C07D 211/58

(54) **Kontinuierliches Verfahren zur Herstellung von 4-Amino-2,2,6,6-tetramethylpiperidin**
Continuous process for the production of 4-amino-2,2,6,6-tetramethylpiperidine
Procédé continu pour la préparation de 4-amino-2,2,6,6-tetramethylpiperidine

(30) Priorität: 30.11.1995 DE 19544599
(43) Veröffentlichungstag der Anmeldung: 04.06.1997
(73) Patentinhaber: Degussa-Hüls Aktiengesellschaft, 60287 Frankfurt am Main (DE)
(72) Erfinder: Jegelka, Udo, Dr., 45657 Recklinghausen (DE); Bachstädter, Gerhard, Dr., 45659 Recklinghausen (DE); Frentzen, Stefan, 46348 Raesfeld (DE); Kreilkamp, Günter, 45770 Marl (DE); Thelen, Gerhard, Dr., 48301 Nottuln (DE)
(74) Vertreter: Olbricht, Gerhard, Dr.

(56) Entgegenhaltungen:
- EP-A- 0 033 529
- EP-A- 0 623 585
- DE-A- 4 442 990
- GB-A- 2 176 473
- US-A- 4 923 992
- PATENT ABSTRACTS OF JAPAN vol. 11, no. 193 (C-430), 20.Juni 1987 & JP 62 016461 A (SUMITOMO CHEM.CO.LTD.), 24.Januar 1987,
- PATENT ABSTRACTS OF JAPAN vol. 10, no. 185 (C-357), 27.Juni 1986 & JP 61 033169 A (SUMITOMO CHEM.CO.LTD.), 17.Dezember 1986,
- DATABASE WPI Week 8820 Derwent Publications Ltd., London, GB; AN 138772 XP002026700 & SU 1 088 304 A (POLMER MAT CHEMICA) 7.November 1987

## Beschreibung

Die vorliegende Erfindung betrifft ein Verfahren zur kontinuierlichen Herstellung von 4-Amino-2,2,6,6-tetramethylpiperidin (TAD) aus Triacetonamin (TAA), Ammoniak und Wasserstoff, in Gegenwart eines oder mehrerer Elemente aus der achten Nebengruppe des Periodensystems als Katalysator, wobei der Katalysator auf einem geeigneten Träger aufgebracht ist und der Träger einen Gehaltsgradienten des Katalysators in Strömungsrichtung aufweist.

Stand der Technik bei großtechnischen Verfahren zur Herstellung von TAD und TAA ist, daß fast ausnahmslos diskontinuierlich gearbeitet wird (z. B. JP 86033169, JP 87016461, GB 2176473, SU 1088304, EP 33529, Deutsche Anmeldung P 44 42 990.8). Ein großer Nachteil der diskontinuierlichen Fahrweise ist, daß sie mit einem regelmäßigen An- und Abfahren der einzelnen Ansätze verbunden ist. Dadurch können nur geringe Raum-Zeit-Ausbeuten bei gleichzeitig erhöhtem Energiebedarf erzielt werden. Zusätzlich erfolgen die Umsetzungen im allgemeinen unter Einsatz von Lösemitteln wie z. B. Alkoholen oder Wasser (z. B. JP 86033169, JP 87016461, GB 2176473, SU 1088304). Als weitere Nachteile dieser Verfahren ergeben sich zusätzliche Aufarbeitungsschritte, wie Kontaktfiltrationen und Kontaktwechsel, sowie das Abdestillieren des Lösemittels. Die zusätzlichen und regelmäßig auftretenden Aufarbeitungsschritte sind kostenintensiv und umweltbelastend. Der entscheidende Nachteil des Batch-Verfahrens ist die Tatsache, daß der Reaktor jedesmal entspannt werden und das überschüssige Ammoniak verdampft werden muß, während bei kontinuierlichen Verfahren das überschüssige Ammoniak zu einem großen Teil im Reaktor verbleiben kann. Die aufzuarbeitende Ammoniak-Menge ist im kontinuierlichen Betrieb damit deutlich geringer.

Bei den Batch-Verfahren werden üblicherweise Kobalt- oder Nickel-Katalysatoren bei Drücken von 10 - 500 bar und Temperaturen von 70 - 200 °C eingesetzt. Mit dem Einsatz pyrophorer Kontakte (EP 33529) ist ein stark nachteiliges Handling verbunden. Ebenfalls nachteilig sind die mit bis zu 10 % pro Ansatz (bezogen auf die verwendete TAA-Menge) (EP 33529, GB 2176473) sehr hohen Katalysatormengen, wodurch sich hohe Katalysator- und Entsorgungskosten ergeben. Weiterhin nachteilig ist, daß sich ausreichende Selektivitäten vielfach nur unter Zusatz von Cokatalysatoren wie z. B. Alkali- oder Erdalkalihydroxiden erzielen lassen (z. B. GB 2176473, SU 1088304).

In EP 623 585 wird die reduktive Aminierung von cyclischen Ketonen in zwei Stufen vorgenommen. Dabei wird in der 1. Stufe mit Ammoniak, katalysiert durch Aktivkohle, ein Ketimin hergestellt, das in der 2. Stufe in Gegenwart eines Kobaltkatalysators, der ein Erdalkalicarbonat oder Lanthanoxid enthält, hydriert wird.

In EP 336 895 wird die reduktive Aminierung einstufig mit Ammoniak und Wasserstoff an einem Hydrierkatalysator, vorzugsweise an einem Platin- oder Palladiumkontakt, durchgeführt. Dabei wird Ammoniak in molarem Unterschuß angewandt.

Der großen Zahl von Batchverfahren steht nur eine geringe Zahl von kontinuierlichen Verfahren zur Herstellung von TAD aus TAA gegenüber. So führt die kontinuierliche Vorgehensweise nach dem Stand der Technik zu keiner wesentlichen Verbesserung gegenüber der diskontinuierlichen Fahrweise. Es wird z. B. gemäß EP 33529 mit pyrophoben Kontakten und Ammoniaküberschüssen von 1 : 10 bis 1 : 50 mol/mol gearbeitet. Die hohen Ammoniakmengen dienen zum einen als Lösemittelersatz und sind zum anderen nötig, um das Gleichgewicht auf die Produktseite zu verschieben und ausreichend hohe Selektivitäten zu erzielen. Nachteilig bleibt somit das Kontakthandling und, bedingt durch den hohen Ammoniakeinsatz, eine ungünstige Energie- und Rohstoffbilanz. Trotz des hohen Ammoniakeinsatzes führen kontinuierliche Verfahren nach dem Stand der Technik zu keiner starken Verbesserung der Raum-Zeit-Ausbeuten: Mit einer LHSV von maximal 0.1 h⁻¹ bezüglich TAA können nur mäßige Durchsätze erreicht werden (LHSV = Liquid-Hourly-Space-Velocity).

Der Erfindung liegt daher die Aufgabe zugrunde, mit einem leistungsfähigen Katalysator- und Kontaktträgersystem die in der Batch-Methodik begründeten Nachteile,
◆ geringe Raum-Zeit-Ausbeute,
◆ schwieriges Kontakthandling,
◆ hoher Ammoniakverbrauch,
◆ aufwendiges Aufarbeiten,
◆ kostenintensives Verfahren,
◆ Umweltbelastung,
welche nach dem Stand der Technik derzeit auch für kontinuierliche Verfahren nicht gelöst sind, au vermeiden und das Verfahren zu verbessern.

Es wurde nun gefunden, daß durch die Verwendung einer Katalysatorpackung mit einem Katalysator, der ein oder mehrere Elemente aus der achten Nebengruppe des Periodensystems mit einem Gehaltsgradienten auf einem geeigneten Träger enthält, in allen Punkten deutliche Verbesserungen gegenüber dem Stand der Technik von diskontinuierlichen und kontinuierlichen Verfahren erzielt werden können.

Gegenstand der Erfindung ist daher ein Verfahren zur kontinuierlichen Herstellung von TAD aus TAA, Ammoniak und Wasserstoff, dadurch gekennzeichnet, daß im Reaktor mindestens zwei Schichten enthalten sind, wobei eine Schicht aus reinem Träger bestehen kann und mindestens eine Schicht ein oder mehrere Elemente der 4. und/oder 5. und/oder 6. Periode der 8. Nebengruppe des Periodensystems als Katalysator auf einem geeigneten Träger enthält und wobei bezüglich jedes Einzelelementes unabhängig voneinander der Katalysatorgehalt vom Reaktoreingang zum Reaktorausgang auf Werte bis 40 % ansteigt.

Durch die Anwendung eines Katalysatorgradienten auf einem geeigneten Träger wird die Leistungsfähigkeit des Systems drastisch erhöht. Die LHSV konnte von bisher 0.1 h⁻¹ bezüglich TAA (EP 33529) auf bis zu 0.6 h⁻¹ bezüglich TAA erhöht werden. Der Gradient ist so angelegt, daß das TAA/Ammoniakgemisch am Reaktoreingang auf einen möglichst geringen Kontaktgehalt trifft. Der Kontaktgehalt und somit die Reaktivität steigern sich bis zum Reaktorausgang. Auf dem Träger werden dabei die Elemente der achten Nebengruppe des Periodensystems eingesetzt. Der Gehalt der Elemente der 4. Periode auf dem Träger steigt unabhängig voneinander vom Reaktoreingang zum Reaktorausgang auf Werte bis 20 %, bevorzugt auf Werte bis 15 % an. Der Gehalt der Elemente der 4. Periode beträgt unabhängig voneinander am Reaktoreingang 0 bis 39 %, vorzugsweise 0 bis 20 % und besonders bevorzugt 2 bis 15 %. Der Gehalt der Elemente der 5. und/oder 6. Periode (sog. Platinmetalle) auf dem Träger steigt unabhängig voneinander vom Reaktoreingang zum Reaktorausgang auf Werte bis 20 %, vorzugsweise auf Werte bis 10 % und besonders bevorzugt auf Werte bis 5 % an. Der Gehalt der Platinmetalle auf dem Träger beträgt unabhängig voneinander am Reaktoreingang 0 bis 19 %, vorzugsweise 0 bis 10 % und besonders bevorzugt 0 bis 5 %.

Dabei werden Trägerpartien mit verschiedenen Katalysatorgehalten geschichtet, so daß es zu einer stufenweisen Änderung des Katalysatorgehaltes kommt. Die Anzahl der Schichten kann dabei zwischen zwei und beliebig vielen gewählt werden. Im letzten Fall wird ein Übergang von einer stufenweisen Änderung des Katalysatorgehaltes zu einem kontinuierlich verlaufenden Gradienten erreicht. Die Elemente werden bevorzugt in metallischer Form verwendet. Kobalt, Nickel, Ruthenium, Palladium und Platin werden bevorzugt eingesetzt. Die Metalle werden per se oder nach Reduktion ihrer Oxide eingesetzt, wobei der Einsatz nicht pyrophorer Kontakte bevorzugt wird. Zur Optimierung des Reaktivitätsgradienten können ein oder auch mehrere Metalle gemischt eingesetzt werden. Bei gleichzeitiger Verwendung von Elementen der 4. Periode und Elementen der Platinmetalle kann der Katalysatorgehalt beim Übergang von den Elementen der 4. Periode zu den Elementen der Platinmetalle in Strömungsrichtung auch abnehmen. Durch die Verwendung eines Gradienten werden hohe Raum-Zeit-Ausbeuten bei vollständigem Umsatz erreicht.

Durch die Abstimmung der Reaktortemperatur auf den Gradienten können Nebenreaktionen weitestgehend unterdrückt und Zersetzungen fast vollständig vermieden werden. So gelingt es, Selektivitäten bis zu ≥ 99 % bezüglich TAA zu erreichen, wenn die Temperaturen bei der aminierenden Hydrierung bis zu 200 °C, vorzugsweise zwischen 80 und 170 °C und insbesondere zwischen 100 °C und 150 °C liegen.

Das Verfahren erlaubt es, daß der Ammoniakverbrauch ohne Qualitätseinbuße von derzeit TAA : Ammoniak bis zu 1 : 50 mol/mol auf 1 : 1 bis 1 : 20 mol/mol, vorzugweise 1 : 1 bis 1 : 10 mol und insbesondere 1 : 3 bis 1 : 5 mol/mol reduziert werden kann.

Hohe Katalysatorstandzeiten werden mit geeigneten Trägermaterialien erreicht. Insbesondere sind hier hydrothermal beständige Aluminiumoxide zu nennen. Es können auch verschiedene geeignete Trägermaterialien geschichtet bzw. gemischt eingesetzt werden.

Die Arbeitsdrücke liegen in den nach dem Stand der Technik üblichen Bereichen bei 10 bis 500 bar, insbesondere bei 20 bis 300 bar.

Auf den Einsatz eines Lösemittels kann bei diesem Verfahren verzichtet werden.

Eine mehrstufige Fahrweise als auch die Verwendung mehrerer in Reihe geschalteter Einzelreaktoren ist möglich, wobei sich dann die Angaben bezüglich des Katalysatorgehaltes auf den Gesamtreaktor, also der Summe der Einzelreaktoren beziehen. Ein einstufiger Prozeß wird jedoch bevorzugt.

Der Vorteil des erfindungsgemäßen Verfahrens beruht auf der wesentlichen Verbesserung der Raum-Zeit-Ausbeute, die durch den hohen Durchsatz erreicht wird. Dies geht einher mit einer deutlichen Verringerung der Herstellkosten, bedingt durch eine einfache Durchführung des kontinuierlichen, inbesondere einstufigen Verfahrens. Durch die hohen Standzeiten werden häufige Katalysatorwechsel vermieden und die Katalysatorkosten drastisch reduziert. Durch das lösemittelfreie Arbeiten und die starke Verringerung des Ammoniakverbrauchs wird die Aufarbeitung stark vereinfacht. Zur Aufarbeitung genügt eine normale Reindestillation, um die bekannten (EP 33529) Ausbeuten und Reinheiten erzielen.

### Beispiel 1:

Ein 0.5 m³-Rieselofen wurde mit 340 kg eines Aluminiumoxid-Trägers gefüllt. Der Träger war mit einem Nickel-Kontakt beschichtet, wobei der Gehalt von Ofenzulauf zum Ofenablauf von 5 % auf 14 % anstieg. TAA und Ammoniak wurden unter einem Wasserstoffdruck von 300 bar durchgesetzt. Bei einer LHSV von 0.4 h⁻¹ TAA und 0.44 h⁻¹ NH₃ (d. h. TAA : NH₃ = 1 : 7.4 mol/mol) und einer Ofentemperatur von 125-145 °C wurde aus 97 %igem TAA 96 %iges TAD erzeugt. Die Aufarbeitung erfolgte destillativ.

### Beispiel 2:

Analog Beispiel 1 wurde an einem 500 ml Rieselofen mit einer LHSV von 0.6 h⁻¹ TAA und 0.4 h⁻¹ NH₃ (d. h. TAA: NH₃ = 1 : 4.5 mol), einem Wasserstoffdruck von 285 bar und einer Ofentemperatur von 130 - 150 °C aus 97 %igem TAA 96 %iges TAD erzeugt. Die Aufarbeitung erfolgte destillativ.

### Beispiel 3:

Ein 500 ml-Rieselofen wurde mit 400 ml eines Aluminiumoxid-Trägers gefüllt. Der Träger war mit einem Nickel-Kontakt beschichtet, wobei der Gehalt von Ofenzulauf zum Ofenablauf von 0 % auf 14 % anstieg. TAA und Ammoniak wurden unter einem Wasserstoffdruck von 285 bar durchgesetzt. Bei einer LHSV von 0.38 h⁻¹ TAA und 0.25 h⁻¹ NH₃ (d. h. TAA:NH₃ = 1 : 4.4 mol) und einer Ofentemperatur von 120 - 145 °C wurde aus 97 %igem TAA 96 %iges TAD erzeugt. Die Aufarbeitung erfolgte destillativ.

## Patentansprüche

1. Verfahren zur kontinuierlichen Herstellung von 4-Amino-2,2,6,6-tetramethylpiperidin (TAD) aus Triacetonamin (TAA), Ammoniak und Wasserstoff,
dadurch gekennzeichnet,
daß im Reaktor mindestens zwei Schichten enthalten sind, wobei eine Schicht aus reinem Träger bestehen kann und mindestens eine Schicht ein oder mehrere Elemente der 4. und/oder 5. und/oder 6. Periode der 8. Nebengruppe des Periodensystems als Katalysator auf einem geeigneten Träger enthält und wobei bezüglich jedes Einzelelementes unabhängig voneinander der Katalysatorgehalt vom Reaktoreingang zum Reaktorausgang auf Werte bis 40 % ansteigt.

2. Verfahren nach Anspruch 1,
dadurch gekennzeichnet,
daß der Katalysatorgehalt für die Elemente der 4. Periode auf dem Träger unabhängig voneinander vom Reaktoreingang zum Reaktorausgang auf Werte bis 20 %, bevorzugt auf Werte bis 15 % ansteigt.

3. Verfahren nach Anspruch 1,
dadurch gekennzeichnet,
daß der Katalysatorgehalt für die Elemente der 4. Periode auf dem Träger unabhängig voneinander am Reaktoreingang 0 bis 39 %, vorzugsweise 0 bis 20 % und besonders bevorzugt 2 bis 15 % beträgt.

4. Verfahren nach Anspruch 1,
dadurch gekennzeichnet,
daß der Katalysatorgehalt für die Elemente der 5. und/oder 6. Periode auf dem Träger unabhängig voneinander vom Reaktoreingang zum Reaktorausgang auf Werte bis 20 %, vorzugsweise auf Werte bis 10 % und besonders bevorzugt auf Werte bis 5 % ansteigt.

5. Verfahren nach Anspruch 1,
dadurch gekennzeichnet,
daß der Katalysatorgehalt für die Elemente der 5. und/oder 6. Periode auf dem Träger unabhängig voneinander am Reaktoreingang 0 bis 19 %. vorzugsweise 0 bis 10 % und besonders bevorzugt 0 bis 5 % beträgt.

6. Verfahren nach mindestens einem der vorhergehenden Ansprüche,
dadurch gekennzeichnet,
daß bei Temperaturen bis zu 200 °C, vorzugsweise zwischen 80 und 170 °C und insbesondere zwischen 100 °C und 150 °C gearbeitet wird.

7. Verfahren nach mindestens einem der vorhergehenden Ansprüche,
dadurch gekennzeichnet,
daß das Molverhältnis TAA : Ammoniak 1 : 1 bis 1 : 20, vorzugsweise 1 : 1 bis 1 : 10 und insbesondere 1 : 3 bis 1 : 5 mol/mol beträgt.

8. Verfahren nach mindestens einem der vorhergehenden Ansprüche,
dadurch gekennzeichnet,
daß als Katalysatormetall Kobalt, Nickel, Ruthenium, Palladium oder Platin oder deren Gemische verwendet werden.

9. Verfahren nach mindestens einem der vorhergehenden Ansprüche,
dadurch gekennzeichnet,
daß bei gleichzeitiger Verwendung von Elementen der 4. Periode und Elementen der Platinmetalle der Katalysatorgehalt bei Übergang von den Elementen der 4. Periode zu den Elementen der Platinmetalle in Strömungsrichtung abnimmt.

## Claims

1. A process for the continuous preparation of 4-amino-2,2,6,6-tetramethylpiperidine (TAD) from triacetoneamine (TAA), ammonia and hydrogen, characterized in that at least two layers are present in the reactor and one layer can consist purely of a support and at least one layer comprises one or more elements of the 4th. and/or 5th. and/or 6th. periods of the 8th. transition group of the Periodic Table as catalyst on a suitable support, where the catalyst content in respect of each individual element rises independently from the reactor inlet to the reactor outlet to values of up to 40%.

2. A process according to claim 1, characterized in that the catalyst content of the elements of the 4th period on the support rises independently of one another from the reactor inlet to the reactor outlet to values of up to 20%, preferably to values of up to 15%.

3. A process according to claim 1, characterized in that at the reactor inlet the catalyst content of the elements of the 4th period on the support is, independently of one another, from 0 to 39%, preferably from 0 to 20% and particularly preferably from 2 to 15%.

4. A process according to claim 1, characterized in that the catalyst content of the elements of the 5th and/or 6th period on the support rises independently of one another from the reactor inlet to the reactor outlet to values of up to 20%, preferably to values of up to 10% and particularly preferably to values of up to 5%.

5. A process according to claim 1, characterized in that at the reactor inlet the catalyst content of the elements of the 5th and/or 6th period on the support is, independently of one another, from 0 to 19%, preferably from 0 to 10% and particularly preferably from 0 to 5%.

6. A process according to at least one of the preceding claims, carried out at temperatures of up to 200°C, preferably from 80 to 170°C and in particular from 100°C to 150°C.

7. A process according to at leat one of the preceding claims, characterized in that the molar ratio TAA:ammonia is from 1:1 to 1:20 mol/mol, preferably from 1:1 to 1:10 mol/mol and in particular from 1:3 to 1:5 mol/mol.

8. A process according to at least one of the preceding claims, characterized in that cobalt, nickel, ruthenium, palladium or platinum or mixtures thereof are used as the catalyst metal.

9. A process according to at least one of the preceding claims, characterized in that, when elements of the 4th period and elements of the platinum metals are used simultaneously, the catalyst content decreases in the flow direction at the transition from the elements of the 4th period to the elements of the platinum metals.

## Revendications

1. Procédé de préparation en continu de 4-amino-2,2,6,6-tétraméthylpipéridine (TAD) à partir de triacétonamine (TAA), d'ammoniac et d'hydrogène,
caractérisé en ce que
le réacteur contient au moins deux couches, une couche pouvant consister en un support pur et au moins un couche contenant un ou plusieurs éléments de la 4^{ème}, et/ou de la 5^{ème} et/ou de la 6^{ème} période du 8^{ème} sous-groupe du système périodique, en tant que catalyseur sur un support convenable, et dans lequel en ce qui concerne chaque élément isolé, indépendamment l'un de l'autre, la teneur du catalyseur de l'entrée dans le réacteur à la sortie du réacteur augmente à des valeurs allant jusqu'à 40 %.

2. Procédé selon la revendication 1,
caractérisé en ce que
la teneur du catalyseur pour les éléments de la 4^{ème} période indépendamment l'un de l'autre, de l'entrée du réacteur à la sortie du réacteur, augmente à des valeurs allant jusqu'à 20 %, de préférence à des valeurs allant jusqu'à 15 %.

3. Procédé selon la revendication 1,
caractérisé en ce que
la teneur du catalyseur pour les éléments de la 4^{ème} période sur le support indépendamment l'un de l'autre, de l'entrée du réacteur à la sortie du réacteur, se situe à des valeurs allant jusqu'à 20 % et d'une manière particulièrement préférée de 2 à 15 %.

4. Procédé selon la revendication 1,
caractérisé en ce que
la teneur du catalyseur pour les éléments de la 5^{ème} et/ou de la 6^{ème} période sur le support, indépendamment l'un de l'autre de l'entrée du réacteur à la sortie du réacteur, augmente à des valeurs allant jusqu'à 20 %, de préférence allant jusqu'à 10 % et d'une manière particulièrement préférée jusqu'à 5 %.

5. Procédé selon la revendication 1,
caractérisé en ce que
la teneur du catalyseur pour les éléments de la 5^{ème} et/ou 6^{ème} période sur le support, indépendamment l'un de l'autre, à l'entrée du réacteur, est de 0 à 19 %, de préférence de 0 à 10 %, et d'une manière particulièrement préférée de 0 à 5 %.

6. Procédé selon au moins une des revendications précédentes,
caractérisé en ce qu'
on opère à des températures allant jusqu'à 200°C, de préférence entre 80 et 170°C, et en particulier entre 100 et 150°C.

7. Procédé selon au moins une des revendications précédentes,
caractérisé en ce que
le rapport molaire TAA/ammoniac est de 1:1 à 1:20, de préférence de 1:1 à 1:10, et en particulier de 1:3 à 1:5 mol/mol.

8. Procédé selon au moins une des revendications précédentes,
caractérisé en ce que
comme métal catalyseur on utilise le cobalt, le nickel, le ruthénium, le palladium ou le platine ou leurs mélanges.

9. Procédé selon au moins une des revendications précédentes,
caractérisé en ce que
simultanément à l'utilisation des éléments de la 4^{ème} période et des éléments des métaux du platine, la teneur du catalyseur diminue par transition des éléments de la 4^{ème} période aux éléments des métaux du platine dans la direction du courant.
